# EUROPÄISCHE PATENTANMELDUNG

(11) **EP 4 620 309 A1**
(43) Veröffentlichungstag der Anmeldung: **24.09.2025**
(21) Anmeldenummer: 24164822.9
(22) Anmeldetag: 20.03.2024
(51) Int. Cl.: A23J 1/00, A23J 1/12, A23J 3/14, A23K 10/38, A23L 7/10, A23L 7/20, A23L 33/185, C12F 3/06, C07K 1/30

(54) **VERFAHREN ZUR EXTRAKTION VON PROTEINEN AUS EINEM PROTEINREICHEN ROHSTOFF**

(71) Anmelder: BDI - BioEnergy International GmbH, 8074 Raaba-Grambach (AT)
(72) Erfinder: Pucher, Peter, 8042 Graz (AT)
(74) Vertreter: Schwarz & Partner Patentanwälte GmbH

(57) **Zusammenfassung**

Die Erfindung betrifft ein Verfahren zur Extraktion von Proteinen aus einem proteinreichen Rohstoff, enthaltend die Schritte
- Behandeln des Rohstoffes (F1) mit einer alkalischen Lösung (L1, L2), wobei eine Mischung (M1, M2) erhalten wird, welche eine feste Phase (R1, R2) und eine proteinreiche flüssige Phase (EXT 1, EXT2), welche die Proteine enthält, umfasst
- Abtrennen der proteinreichen flüssigen Phase (EXT 1, EXT2) von der festen Phase (R1, R2),
- Absenken des pH-Wertes in der proteinreichen flüssigen Phase (EXT 1, EXT 2, M3), wobei die Proteine ausgefällt werden und eine weitere Mischung (M4, M5) erhalten wird, welche eine weitere flüssige Phase und eine proteinreiche feste Phase umfasst,
- Abtrennen der ausgefällten Proteine aus der Mischung (M4, M5) zur Gewinnung eines Proteinextrakts (R4).

Das erfindungsgemäße Verfahren ist dadurch gekennzeichnet, dass zum Absenken des pH-Wertes in der proteinreichen flüssigen Phase (EXT 1, EXT 2, M3) ein kohlendioxid-hältiges Gas (G3) eingesetzt wird.

## Beschreibung

Die vorliegende Erfindung befasst sich mit einem Verfahren zur Extraktion von Proteinen aus einem proteinreichen Rohstoff, insbesondere aus einem Rückstand der Bierproduktion, im Speziellen aus Biertreber.

Angesichts der Notwendigkeit, die immer stärker wachsende Bevölkerung der Welt zu ernähren, werden umfangreiche Forschungen und Arbeiten zur Entwicklung von Nahrungsmitteln aus nährstoffreichen Abfallprodukten verschiedenster industrieller Prozesse betrieben.

Die vorliegende Erfindung beschäftigt sich insbesondere mit der Gewinnung von einem an Proteinen reichen Produkt aus einem proteinreichen Abfallprodukt der Lebensmittelindustrie, wie z.b. Reststoffe aus Bäckereien, der Obstverarbeitung, der Süßwarenproduktion, und im speziellen Abfallprodukte aus Brennereien und Brauereien.

Im Folgenden wird insbesondere auf die Gewinnung eines proteinreichen Produkts aus Biertreber eingegangen, doch gelten die Ausführungen analog auch für andere proteinreiche Roh- bzw. Abfallstoffe.

In der EU fallen in Brauereien jährlich ca. 3,4 Millionen Tonnen an Abfallprodukten an, wovon Biertreber ("Brewer's spent grain, BSG") ungefähr 85% ausmachen. Der Gehalt an Proteinen in Biertreber kann beispielsweise 19 - 30% betragen (Wen et al., A Mini-Review on Brewer's Spent Grain Protein, J. of Food Science 84 (12) 2019, 3330-3340).

Andere proteinreiche Abfallprodukte aus Bierbrauereien sind Abfallhefe (Spent Yeast) und Abfallhopfen (Hot Trub) (Rodriguez et al., Protein recovery from brewery solid wastes, J. Foodchem, 2023 (407), 134810).

Die Extraktion der Proteine aus solchen proteinreichen Rohstoffen kann auf verschiedene Arten erfolgen, wobei im Fall von Biertreber insbesondere die Methoden der alkalischen Extraktion, der Extraktion mit organischen Lösungsmitteln (insbesondere Ethanol), enzymatische Extraktion, alle gegebenenfalls durch Vorbehandlung mit Ultraschall unterstützt, genannt werden können.

Weiteres Hintergrundwissen zur Verarbeitung von Biertreber, insbesondere der Gewinnung von Proteinen daraus, ist der WO 2021/028405 und der darin zitierten Literatur sowie auch Vieira et al., Valuation of brewer's spent grain using a fully recyclable integrated process for extraction of proteins and arabinoxylans, Industrial Crops and Products 52 (2014) 136-143 zu entnehmen.

Die vorliegende Erfindung beschäftigt sich mit der alkalischen Extraktion von Proteinen aus den genannten proteinreichen Rohstoffen, gefolgt von einer Ausfällung der Proteine durch Absenken des pH-Werts.

Die Extraktion von Proteinen aus proteinreichen Rohstoffen bzw. Biertreber - einem Rückstand der Bierproduktion - erfolgt dabei üblicherweise mit Hilfe von alkalischen Lösungen wie z.B. Natronlauge oder Kalilauge bei einem pH-Wert von über 9-10. Die anschließende Gewinnung der Proteine erfolgt - neben anderen Methoden - üblicherweise durch Absenken des pH-Wertes auf den isoelektrischen Punkt. Dadurch flocken die Proteine aus und können z.B. über Filtration oder Zentrifugation abgetrennt werden. Beispielhaft für das alkalische Extrakt aus Biertreber muss ein pH-Wert unter pH 4 eingestellt werden.

Ein Nachteil dieser Methode ist, dass für den Wechsel vom ursprünglich stark alkalischen pH-Bereich auf einen stark sauren pH-Wert ein nicht unerheblicher Anteil an Säure benötigt wird. Werden dazu mineralische Säuren wie Salzsäure, Phosphorsäure oder Schwefelsäure eingesetzt, so werden dadurch auch Fremdelemente wie Chlor, Phosphor oder Schwefel eingebracht, welche die Eigenschaften, Farbe und Geschmack vom gefällten Protein-Konzentrat beeinflussen können. Es wird daher in weiterer Folge noch eine Wäsche notwendig, um die gebildeten Salze und Reste der Säure zu entfernen.

Werden organische Säuren wie z.B. Zitronensäure eingesetzt, so sind diese üblicherweise teuer, stammen oft von nicht biogenen Quellen und beeinflussen ebenfalls den Geschmack des finalen Konzentrats.

Die vorliegende Erfindung stellt sich zur Aufgabe, die Nachteile der bekannten Verfahren zur Gewinnung von Proteinen aus proteinreichen Rohstoffen, insbesondere Biertreber, zu überwinden, und ein verbessertes Verfahren zur Verfügung zu stellen.

Diese Aufgabe wird mit einem Verfahren gelöst, enthaltend die Schritte
- Behandeln des Rohstoffes mit einer alkalischen Lösung , wobei eine Mischung erhalten wird, welche eine feste Phase und eine proteinreiche flüssige Phase, welche die Proteine enthält, umfasst
- Abtrennen der proteinreichen flüssigen Phase von der festen Phase,
- Absenken des pH-Wertes in der proteinreichen flüssigen Phase, wobei die Proteine ausgefällt werden und eine weitere Mischung erhalten wird, welche eine weitere flüssige Phase und eine proteinreiche feste Phase umfasst,
- Abtrennen der ausgefällten Proteine aus der Mischung zur Gewinnung eines Proteinextrakts,
und welches dadurch gekennzeichnet ist, dass zum Absenken des pH-Wertes in der proteinreichen flüssigen Phase ein kohlendioxid-hältiges Gas eingesetzt wird.

### KURZE BESCHREIBUNG DER FIGUREN

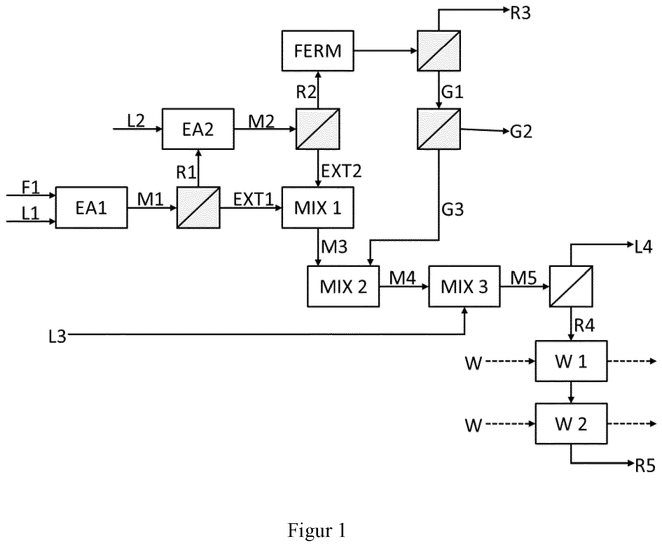

Figur 1 zeigt schematisch als Flussdiagramm eine bevorzugte Ausführungsform des erfindungsgemäßen Verfahrens.

### DETAILLIERTE BESCHREIBUNG DER ERFINDUNG

Es wurde überraschenderweise gefunden, dass durch den zumindest teilweisen Ersatz der oben beschriebenen anorganischen oder organischen Säuren zum Ausfällen der nach alkalischer Extraktion erhaltenen in der flüssigen Phase vorliegenden Proteine durch Kohlendioxid die bekannten Verfahren in mehrfacher Hinsicht verbessert werden können:
Zum einen kann der Bedarf an Säuren/Chemikalien reduziert werden, was auch eine Kostenreduktion bedeutet. Weiters kann der Anfall an schwer weiterverarbeitbaren Reaktionsprodukten wie z.B. Salzen, reduziert werden. Ebenfalls können die beschriebenen Nachteile wie unvorteilhafter Geschmack des erhaltenen Proteinextrakts etc. verringert bzw. auch der Aufwand für eine Wäsche verringert werden.

Da mit dem Einsatz von Kohlendioxid allein eine Absenkung des pH-Wertes lediglich auf etwa pH 6,5 möglich ist, wird das Kohlendioxid im Schritt des Ausfällens in an sich bekannter Weise durch eine zur Erreichung eines niedrigeren pH-Wertes notwendige Restmenge an anorganischen und/oder organischen Säuren ergänzt.

In einer bevorzugten Ausführungsform des erfindungsgemäßen Verfahrens stammt das zum Ausfällen der Proteine eingesetzte kohlendioxid-hältige Gas aus einer biogenen Quelle, insbesondere aus einer Fermentation.

Eine besonders bevorzugte Ausführungsform ist dadurch gekennzeichnet, dass das kohlendioxid-hältige Gas aus einer Fermentation der nach dem Behandeln des Rohstoffes mit der alkalischen Lösung erhaltenen festen Phase stammt. Bei der Reaktion der Lauge mit dem Rohstoff wird im Rohstoff enthaltenes biogenes CO₂ gebunden. Bereits dadurch wird ein Beitrag zur Reduktion klima-schädlicher Gase geleistet.

Die nach der alkalischen Extraktion resultierende feste Phase kann mittels Fermentation zur Gewinnung von Biogas aufgearbeitet werden. Das bei der Fermentation entstehende Kohlendioxid kann nunmehr zur Ausfällung der Proteine nach der alkalischen Extraktion eingesetzt werden. Durch diese interne Kreislaufführung kann eine weitere Einsparung und Effizienzsteigerung des Gesamtverfahrens (kein Einsatz von externem Kohlendioxid, verkürzte Transportwege) erzielt werden.

Manche Stufen des erfindungsgemäßen Verfahrens können zur weiteren Steigerung der Effizienz wiederholt durchgeführt und miteinander verbunden werden:
So kann die nach dem Behandeln des Rohstoffes mit der alkalischen Lösung erhaltene feste Phase erneut mit einer alkalischen Lösung behandelt werden, wobei eine weitere Mischung erhalten wird, welche eine weitere feste Phase, und eine weitere flüssige Phase, welche einen weiteren Anteil an Proteinen enthält, umfasst. Die weitere, Proteine enthaltende flüssige Phase kann mit der ersten erhaltenen, Proteine enthaltenden flüssigen Phase vor dem Ausfällen vereinigt werden.

Auch die Stufe der Absenkung des pH-Wertes zur Ausfällung der Proteine kann wiederholt werden. Beispielsweise kann in einer ersten Stufe des Absenkens das kohlendioxid-hältige Gas zugeführt und in einer weiteren Stufe die zum Erreichen eines niedrigeren pH-Wertes notwendige Menge an organischen und/oder anorganischen Säuren, ggf. auch in Mischung mit weiterem Kohlendioxid, zugeführt werden.

### DETAILLIERTE BESCHREIBUNG DER FIGUR

Gemäß Figur 1 wird in einer bevorzugten Ausführungsform der vorliegenden Erfindung ein proteinreicher Rohstoff, z.B. Biertreber, als Feed F 1 einem ersten Extraktionsaggregat EA1 zugeführt. Dem Extraktionsaggregat EA1 wird ein alkalisches flüssiges Extraktionsmittel L1 zugeführt.

Die resultierende erste Mischung M1 umfasst eine flüssige, einen hohen Anteil an Proteinen enthaltende Phase EXT1 und eine feste Phase R1. Diese beiden Phasen werden in an sich bekannter Weise voneinander getrennt.

In der Ausführungsform gemäß Figur 1 wird die feste Phase R1 in einem weiteren Extraktionsaggregat EA2 mit weiterem alkalischen oder neutralem (z.B. Wasser) Extraktionsmittel L2 behandelt, wobei eine weitere Mischung M2, deren flüssige Phase EXT 2 einen weiteren Anteil an Proteinen enthält, erhalten wird.

Die beiden Phasen R2 und EXT2 der Mischung M2 werden erneut in an sich bekannter Weise voneinander getrennt.

Die beiden erhaltenen proteinreichen flüssigen Phasen EXT1 und EXT2 werden in einem ersten Mischer MIX1 zu einer Mischung M3 gemischt.

Die Mischung M3 wird in einem weiteren Mischer MIX2 mit kohlendioxidhältigem Gas G3 versetzt und damit angesäuert.

Das kohlendioxid-hältige Gas G3 stammt in der Ausführungsform gemäß Figur 1 aus einer Fermentation FERM des festen Rückstandes R2 zur Herstellung von Biogas. Das in der Fermentation entstandene Rohbiogas G1 wird auf an sich bekannte Weise zu einem methanreichen Strom G2 und dem kohlendioxidreichen Strom G3 aufgetrennt. Der feste Rückstand der Fermentation R3 wird aus dem Verfahren ausgetragen.

Zur weiteren Ansäuerung kann die nach Versetzen der Mischung M3 mit dem kohlendioxidhältigen Gas G3 erhaltene weitere Mischung M4 beispielsweise in einem weiteren Mischer MIX3 mit anorganischer und/oder organischer Säure L3 bis zum Erreichen des gewünschten pH-Wertes versetzt werden.

Die erhaltene Mischung M5 enthält die durch die Ansäuerung ausgefällten Proteine in einer festen Phase R4. Diese proteinreiche Phase R4 kann erneut in an sich bekannter Weise von der entstandenen flüssigen Phase L4 abgetrennt werden und gegebenenfalls in zwei Waschschritten W1 und W2 mit Waschmedium W gewaschen werden. Es wird ein gewaschenes Proteinextrakt R5 erhalten, welches einer weiteren Verwendung zugeführt werden kann.

### BEISPIELE

### Beispiel 1:

50,05g von einem alkalisch mit NaOH extrahiertem Extrakt M3 mit einem pH-Wert von pH 11,9 wird mit 4,5g 1M HCl auf pH 2,1 eingestellt. 53,697g der Lösung werden in ein Zentrifugenröhrchen überführt und der entstandene Niederschlag 5 min bei 4000 rpm abgetrennt und feucht mit 5,478g ausgewogen. Nach Trocknung bei 105°C über Nacht wurden 0,53g trockenes Konzentrat erhalten.

### Beispiel 2:

50,05g von einem alkalisch mit NaOH extrahiertem Extrakt M3 mit einem pH-Wert von pH 11,5 wird mit CO₂ begast, bis sich ein pH-Wert von ca. pH 6,5eingestellt hat. Die Lösung wird in ein Zentrifugenröhrchen überführt und 5 min bei 4000 rpm zentrifugiert. Dabei bildet sich noch kein Niederschlag. 48,06g der Lösung werden wieder in ein gerührtes Becherglas mit pH-Messung überführt. Durch Zugabe von 3,76g 1M HCl wird ein pH-Wert von pH 2,3 eingestellt. 51,82g der Lösung werden in ein Zentrifugenröhrchen überführt und der entstandene Niederschlag 5 min bei 4000 rpm abgetrennt. Die feuchte Auswaage wurde mit 3,52g ausgewogen.

Nach Trocknung bei 105°C über Nacht wurden 0,499g trockenes Konzentrat erhalten.

Die Einsparung an Säure beim Beispiel 2 zum Beispiel 1 liegt bei etwa 11-12% bezogen auf getrocknetes Produkt. Das getrocknete Produkt von Beispiel 2 schmeckt angenehm mildsäuerlich und nur leicht salzig.

## Patentansprüche

1. Verfahren zur Extraktion von Proteinen aus einem proteinreichen Rohstoff, enthaltend die Schritte
- Behandeln des Rohstoffes (F1) mit einer alkalischen Lösung (L1, L2), wobei eine Mischung (M1, M2) erhalten wird, welche eine feste Phase (R1, R2) und eine proteinreiche flüssige Phase (EXT 1, EXT2), welche die Proteine enthält, umfasst
- Abtrennen der proteinreichen flüssigen Phase (EXT 1, EXT2) von der festen Phase (R1, R2),
- Absenken des pH-Wertes in der proteinreichen flüssigen Phase (EXT 1, EXT 2, M3), wobei die Proteine ausgefällt werden und eine weitere Mischung (M4, M5) erhalten wird, welche eine weitere flüssige Phase und eine proteinreiche feste Phase umfasst,
- Abtrennen der ausgefällten Proteine aus der Mischung (M4, M5) zur Gewinnung eines Proteinextrakts (R4),
**dadurch gekennzeichnet, dass** zum Absenken des pH-Wertes in der proteinreichen flüssigen Phase (EXT 1, EXT 2, M3) ein kohlendioxid-hältiges Gas (G3) eingesetzt wird.

2. Verfahren gemäß Anspruch 1, **dadurch gekennzeichnet, dass** das kohlendioxid-hältige Gas (G3) aus einer biogenen Quelle, insbesondere aus einer Fermentation, stammt.

3. Verfahren gemäß Anspruch 2, **dadurch gekennzeichnet, dass** das kohlendioxid-hältige Gas (G3) aus einer Fermentation der nach dem Behandeln des Rohstoffes (F1) mit der alkalischen Lösung (L1, L2) erhaltenen festen Phase (R1, R2) stammt.

4. Verfahren gemäß einem der vorhergehenden Ansprüche, **dadurch gekennzeichnet, dass** der proteinreiche Rohstoff ein Rückstand der Bierproduktion, insbesondere Biertreber, ist.
